(19) Europäisches Patentamt
European Patent Office
Office européen des brevets

(11) Veröffentlichungsnummer: **0 037 938**
**A1**

(12) **EUROPÄISCHE PATENTANMELDUNG**

(21) Anmeldenummer: **81102277.1**

(22) Anmeldetag: **26.03.81**

(51) Int. Cl.³: **C 07 D 263/58**, C 07 D 277/68, A 01 N 43/76, A 01 N 43/78

(30) Priorität: **07.04.80 JP 45356/80**

(43) Veröffentlichungstag der Anmeldung: **21.10.81 Patentblatt 81/42**

(84) Benannte Vertragsstaaten: **BE CH DE FR GB IT LI NL**

(71) Anmelder: **NIHON TOKUSHU NOYAKU SEIZO K.K., No.8, 2-chome, Nihonbashi Muromachi, Chuo-Ku, Tokyo (JP)**

(72) Erfinder: **Kuyama, Shinpei, 3-21-39, Meguro Meguro-ku, Tokyo (JP)**
Erfinder: **Aya, Masahiro, 2-844, Suzuki-Cho Kodaira-shi, Tokyo (JP)**
Erfinder: **Saito, Junichi, 3-7-12, Osawa Mitaka-shi, Tokyo (JP)**

(74) Vertreter: **Gremm, Joachim, Dr. et al, c/o Bayer AG, Zentralbereich Patente Marken und Lizenzen, D-5090 Leverkusen, Bayerwerk (DE)**

(54) **Substituierte Acetanilide, Verfahren zu ihrer Herstellung und ihre Verwendung als Herbizide.**

(57) Neue substituierte Acetanilide der allgemeinen Formel

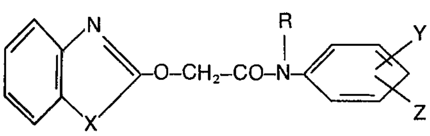

(I)

in der X ein Sauerstoffatom oder ein Schwefelatom ist, R ein Alkylrest mit 1 bis 4 C-Atomen ist und Y und Z, die gleich oder verschieden sein können, jeweils für ein Wasserstoffatom, einen Alkoxyrest mit 1 bis 4 C-Atomen, einen Halogenalkylrest oder einen Phenoxyrest stehen oder gemeinsam eine Trimethylengruppe bilden mit der Maßgabe, daß Y und Z nicht beide Wasserstoffatome sind,
ein Verfahren zu ihrer Herstellung und ihre Verwendung als Herbizide.

EP 0 037 938 A1

0037938

NIHON TOKUSHU NOYAKU SEIZO K.K.

Bi/Di
I a

## Substituierte Acetanilide, Verfahren zu ihrer Herstellung und ihre Verwendung als Herbizide

Die Erfindung betrifft gewisse neue substituierte Acetanilide, ein Verfahren zu ihrer Herstellung und ihre Verwendung als Herbizide.

In der japanischen Auslegeschrift 154762/1979 wird festgestellt, daß substituierte Carbonsäureamide der allgemeinen Formel

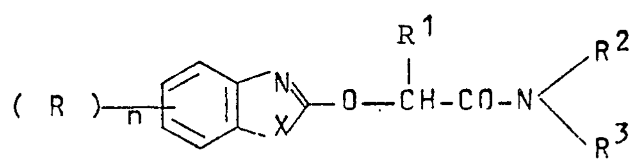

(IV),

in der n für 1, 2, 3 oder 4 steht,
jeder Rest R einzeln für Wasserstoff, Halogen, Alkyl, Halogenalkyl, Alkoxy, Halogenalkoxy, Alkylthio, Halogenalkylthio, Amino, Alkylamino, Dialkylamino, Nitro, Cyan oder Alkoxycarbonyl steht oder zwei Reste R gemeinsam für Methylendioxy, Dichlormethylendioxy oder Difluormethylendioxy stehen, $R^1$ für Wasserstoff oder Alkyl steht, $R^2$ und $R^3$ gleich oder verschieden sein können und jeweils für Wasserstoff, Alkyl, Alkenyl, Alkinyl, Aralkyl, Cycloalkyl, Aryl oder eine gegebenenfalls substituierte heterocyclische Gruppe stehen oder gemeinsam mit dem Stickstoffatom, an das sie gebunden sind, eine substituierte oder unsubstituierte, gegebenenfalls teilweise ungesättigte

monocyclische oder bicyclische Gruppe, die ein oder mehrere weitere Heteroatome bilden und gegebenenfalls kondensierte Benzolringe enthalten kann, und X Sauerstoff oder Schwefel ist, herbizide Wirkung aufweisen.

Gegenstand der Erfindung sind neue substituierte Acetanilide der allgemeinen Formel

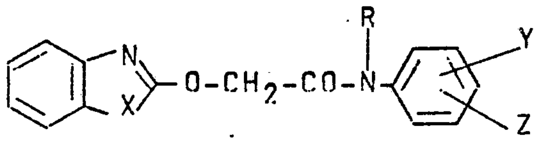

(I)

in der X ein Sauerstoffatom oder ein Schwefelatom ist, R ein Alkylrest mit 1 bis 4 C-Atomen ist und Y und Z, die gleich oder verschieden sein können, jeweils für ein Wasserstoffatom, einen Alkoxyrest mit 1 bis 4 C-Atomen, einen Halogenalkylrest oder einen Phenoxyrest stehen oder gemeinsam eine Trimethylengruppe bilden mit der Maßgabe, daß Y und Z nicht beide Wasserstoffatome sind.

Die Erfindung umfaßt ferner die Herstellung von substituierten Acetaniliden der Formel (I) nach einem Verfahren, das dadurch gekennzeichnet ist, daß man ein 2-Halogenbenzazol der allgemeinen Formel

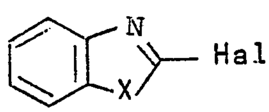

(II),

in der X die vorstehend genannte Bedeutung hat und Hal ein Halogenatom ist, mit einer Verbindung der allgemeinen Formel

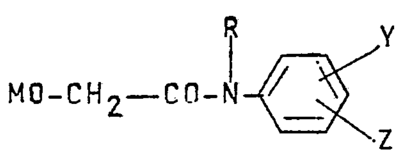

(III),

in der R, Y und Z die vorstehend genannten Bedeutungen haben und M ein Wasserstoffatom oder Alkalimetallatom ist, umsetzt.

Die substituierten Acetanilide der vorstehenden Formel (I) werden zwar allgemein von der vorstehenden Formel (IV)

umfaßt, jedoch findet sich in der vorstehend genannten japanischen Patentanmeldung keine Beschreibung der Verbindungen gemäß der Erfindung.

Es wurde gefunden, daß die substituierten Acetanilide der Formel (I) eine überraschend überlegene herbizide Aktivität im Vergleich zu bekannten analogen Verbindungen aufweisen. Insbesondere haben die Verbindungen gemäß der Erfindung eine herbizide Aktivität, die im Vergleich zu den in der genannten japanischen Patentanmeldung beschriebenen analogen Verbindungen besonders gut hinsichtlich der Spezifität ist. Die Erfindung stellt somit einen bedeutenden technischen Fortschritt dar.

Von den Verbindungen der Formel (I) werden solche bevorzugt, in denen R ein Methyl-, Äthyl-, n-Propyl- oder Isopropylrest oder ein n-Butyl-, Isobutyl-, sek.-Butyl- oder t-Butylrest ist und Y und Z jeweils für ein Wasserstoffatom, einen Methoxy-, Äthoxy-, n-Propoxy-, Isopropoxy-, n-Butoxy-, Isobutoxy-, sek.-Butoxy- oder t-Butoxyrest, einen Phenoxyrest oder eine Monohalogenmethyl-, Monohalogenäthyl-, Dihalogenmethyl-, Dihalogenäthyl-, Trihalogenmethyl- oder Trihalogenäthylgruppe stehen, wobei der Halogensubstituent bzw. die Halogensubstituenten Fluor, Chlor, Brom oder Jod sind, oder worin Y und Z gemeinsam einen Trimethylenrest bilden.

Das Verfahren zur Herstellung der Verbindungen (I) wurde vorstehend beschrieben. Beispiele der als Ausgangsmaterialien verwendeten 2-Halogenbenzazole der Formel (II) sind 2-Chlorbenzothiazol, 2-Chlorbenzoxazol und ihre entsprechenden 2-Bromverbindungen.

Beispiele der ebenfalls als Ausgangsmaterialien verwendeten Verbindungen der Formel (III) sind 2'-Trifluormethyl-N-methylhydroxyacetanilid, 3'-Trifluormethyl-N-methylhydroxyacetanilid, 4'-Trifluormethyl-N-methylhydroxyacetanilid,

3',5'-Bis-trifluormethyl-N-methylhydroxyacetanilid, 2'-Methoxy-N-methylhydroxyacetanilid, 3'-Methoxy-N-methylhydroxyacetanilid, 4'-Methoxy-N-methylhydroxyacetanilid, 3'-Isopropoxy-N-methylhydroxyacetanilid, 3'-Phenoxy-N-methylhydroxyacetanilid, N-5-Indanyl-N-methylhydroxyacetanilid und ihre Alkalisalze.

Bei Verwendung von 2-Chlorbenzothiazol und 3'-Trifluormethyl-N-methyl-hydroxyacetanilid als Ausgangsmaterialien kann das Herstellungsverfahren durch die folgende Gleichung dargestellt werden:

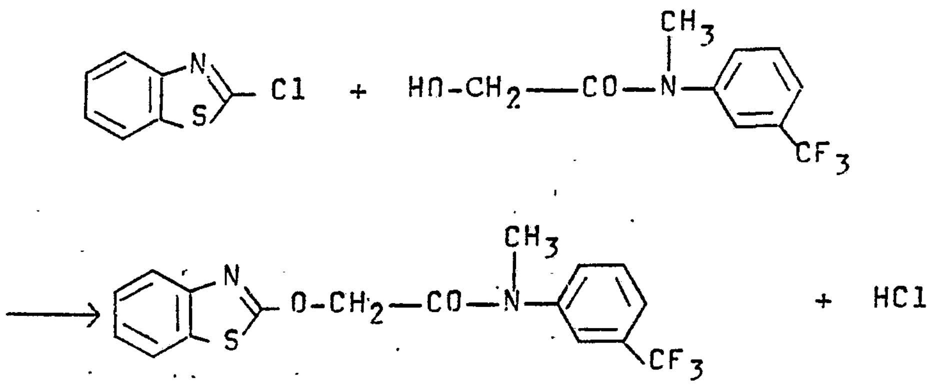

Das Verfahren gemäß der Erfindung wird vorzugsweise in Gegenwart eines Lösungsmittels oder Verdünnungsmittels durchgeführt. Zu diesem Zweck können beliebige inerte Lösungs- oder Verdünnungsmittel verwendet werden. Beispiele solcher Lösungs- und Verdünnungsmittel sind Wasser, aliphatische, alicyclische und aromatische Kohlenwasserstoffe (die gegebenenfalls chloriert sein können), z.B. Hexan, Cyclohexan, Petroläther, Ligroin, Benzol, Toluol, Xylol, Methylenchlorid, Chloroform, Tetrachlorkohlenstoff, Äthylenchlorid, Trichloräthylen oder Chlorbenzol, Äther, z.B. Diäthyläther, Methyläthyläther, Diisopropyläther, Dibutyläther, Propylenoxid, Dioxan oder Tetrahydrofuran, Ketone, z.B. Aceton, Methyläthylketon, Methylisopropylketon und Methylisobutylketon, Nitrile, z.B. Acetonitril, Propionitril oder Acrylnitril, Alkohole, z.B. Methanol, Äthanol, Isopropanol, Butanol oder Äthylenglykol, Ester, z.B. Äthylacetat oder Amylacetat, Säureamide, z.B. Dimethylformamid oder Dimethylacetamid, Sulfone und Sulfoxide, z.B. Dimethylsulfoxid oder Sulfolan, und organische Basen, z.B. Pyridin.

Das Verfahren gemäß der Erfindung kann in Gegenwart eines säurebindenden Mittels durchgeführt werden, insbesondere wenn M in der Formel (III) Wasserstoff ist. Beispiele solcher säurebindenden Verbindungen sind die üblicherweise verwendeten Mittel, z.B. die Hydroxide, Carbonate, Bicarbonate und Alkoholate von Alkalimetallen, und tertiäre Amine, z.B. Triäthylamin, Diäthylanilin oder Pyridin.

Das Verfahren gemäß der Erfindung kann über einen weiten Bereich von Temperaturen durchgeführt werden. Im allgemeinen wird es bei einer Temperatur zwischen -20°C und dem Siedepunkt des Reaktionsgemisches, vorzugsweise zwischen 0°C und 100°C, durchgeführt. Als Reaktionsdruck wird vorzugsweise Normaldruck angewandt, jedoch kann, falls dies zweckmäßig ist, auch bei erhöhtem oder vermindertem Druck gearbeitet werden.

Da die Verbindungen gemäß der Erfindung nur eine geringe Toxizität für Warmblüter und gute Selektivität hinsichtlich der angebauten Pflanzen haben, d.h. bei den üblicherweise angewandten Konzentrationen keine Phytotoxizität zeigen, können sie günstig als Herbizide zur Unkrautbekämpfung verwendet werden. Unter "Unkräutern" im weitesten Sinne sind Pflanzen zu verstehen, die an Stellen wachsen, wo sie nicht erwünscht sind.

Die Verbindungen gemäß der Erfindung sind ausgezeichnet hinsichtlich ihrer Sicherheit, zeigen überlegene herbizide Aktivität und weisen ein breites herbizides Spektrum auf.

Die aktiven Verbindungen gemäß der Erfindung können beispielsweise zur Bekämpfung der folgenden Pflanzen verwendet werden: Dikotyledon-Unkräuter der Gattungen Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Rorippa, Rotala, Lindernia,

Lamium, Veronica, Abutilon, Emex, Datura, Viola, Galeopsis, Papaver, Centaurea und Solanum, und Monokotyledon-Unkräuter der Gattungen Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus und Apera.

Die aktiven Verbindungen gemäß der Erfindung können beispielsweise als selektive Herbizide in den folgenden Kulturen verwendet werden:
Dikotyledonkulturen der Gattungen Gossypium, Glycina, Beta, Daucus, Phaseolus, Pisum, Solanum, Linum, Ipomoea, Vicia, Nicotiana, Lycopersicon, Arachis, Brassica, Lactuca, Cucumis und Cucurbita, und
Monokotyledonkulturen der Gattungen Oryza, Zea, Triticum, Hordeum, Avena, Secale, Sorghum, Panicum, Saccharum, Ananas, Asparagus und Allium.

Die Anwendung der aktiven Verbindungen gemäß der Erfindung ist jedoch keineswegs auf diese Gattungen begrenzt, sondern umfaßt auch andere Pflanzen in der gleichen Weise.

In Abhängigkeit von den Konzentrationen können die Verbindungungen zur totalen Bekämpfung von Unkräutern, z.B. auf Industriegelände und Bahnkörpern und auf Wegen und Plätzen mit oder ohne Bäume verwendet werden. Ebenso können die Verbindungen zur Unkrautbekämpfung in perennierenden Kulturen, z.B. Aufforstungen, Zierbaumpflanzungen, Obstgärten, Weinbergen und -gärten, Zitruswäldern, Nußbaumpflanzungen, Bananenplantagen, Kaffeeplantagen, Teeplantagen, Gummiplantagen, Ölpalmenplantagen, Kakaoplantagen, Weichfruchtplantagen und Hopfenfeldern und zur selektiven Unkrautbekämpfung in Jahreskulturen verwendet werden.

Es ist darauf hinzuweisen, daß die Verbindungen gemäß der

Erfindung eine überlegene selektive Bekämpfungswirkung insbesondere bei Verwendung als Bodenbehandlungsmittel vor dem Auflaufen und als Pflanzen-und Bodenbehandlungsmittel für Reisfeldunkräuter aufweisen. Beispielsweise zeigen sie herbizide Aktivität gegen die folgenden Reisfeld-Unkräuter, ohne daß sie irgendwelche Phytotoxizität gegen Reispflanzen aufweisen: Die Dikotyledon-Unkräuter Rotala indica Koehne, Lindernia procumbens Philcox, Ludwigia prostrata Roxburgh, Potamogeton distinctus A. Bennett und Elatine triandra Schkuhr, und die Monokotyledon-Unkräuter Echinochloa crus-galli Beauvois, Monochoria vaginalis Presl, Eleocharis aciacicularis L., Eleocharis kuroguwai Ohwi, Cyperus difformis L., Cyperus serotinus Rottboell, Sagittaria pigmaea Miquel, Alisma canaliculatum A. Braun et Bouché und Scirpus juncoides Roxburgh var.
Die Wirkstoffe können in übliche Präparate, z.B. Lösungen, Emulsionen, Suspensionen, Pulver, Schäume, Granulat, natürliche und synthetische Materialien, die mit den Wirkstoffen imprägniert sind, und sehr feine Kapseln in polymeren Substanzen umgewandelt werden.

Diese Präparate können in bekannter Weise hergestellt werden, beispielsweise durch Mischen der Wirksubstanzen mit Streckmitteln, d.h. flüssigen oder festen Verdünnungsmitteln oder Trägern gegebenenfalls unter Verwendung von oberflächenaktiven Mitteln, d.h. Emulgatoren und/oder Dispergiermitteln und/oder schaumbildenden Mitteln. Bei Verwendung von Wasser als Streckmittel können beispielsweise auch organische Lösungsmittel als Hilfslösungsmittel verwendet werden.

Als flüssige Streck- und Verdünnungsmittel oder Träger, insbesondere als Lösungsmittel, eignen sich hauptsächlich aromatische Kohlenwasserstoffe, z.B. Xylol, Toluol oder Alkylnaphthaline, chlorierte aromatische oder chlorierte aliphatische Kohlenwasserstoffe, z.B. Chlorbenzole, Chloräthylene oder Methylenchlorid, aliphatische oder alicyclische Kohlenwasserstoffe, z.B. Cyclohexan oder Paraffine,

z.B. Mineralölfraktionen, Alkohole, z.B. Butanol oder Glykol, sowie ihre Äther und Ester, Ketone, z.B. Aceton, Methyläthylketon, Methylisobutylketon oder Cyclohexanon, oder stark polare Lösungsmittel, z.B. Dimethylformamid und Dimethylsulfoxid, sowie Wasser.

Als feste Träger können gemahlene natürliche Minerale, z.B. Kaoline, Tone, Talkum, Kreide, Quarz, Attapulgit, Montmorillonit oder Diatomeenerde, und gemahlene synthetische Minerale, z.B. hochdisperse Kieselsäure, Aluminiumoxid und Silicate, verwendet werden. Als feste Träger für Granulat können zerkleinerte und fraktionierte natürliche Gesteine, z.B. Kalzit, Marmor, Bimsstein, Sepiolith und Dolomit, sowie synthetisches Granulat von anorganischen und organischen Mehlen, und Granulat von organischem Material, z.B. Sägemehl, Kokosnußschalen, Maiskolben und Tabakstengel, verwendet werden.

Als Emulgatoren und/oder Schaumbildner können nichtionogene und anionaktive Emulgatoren, z.B. Polyoxyäthylen-Fettsäureester, Polyoxyäthylen-Fettalkoholäther, z.B. Alkylarylpolyglykoläther, Alkylsulfonate, Alkylsulfate, Arylsulfonate sowie Produkte der Hydrolyse von Albumin, verwendet werden. Beispiele geeigneter Dispergiermittel sind Ligninsulfitablaugen und Methylcellulose.

Haftmittel, z.B. Carboxymethylcellulose und natürliche und synthetische Polymerisate in Form von Pulver, Granulat oder Latices, z.B. Gummiarabicum, Polyvinylalkohol und Polyvinylacetat, können in den Präparaten verwendet werden.

Es ist möglich, farbgebende Stoffe, z.B. anorganische Pigmente, z.B. Eisenoxid, Titanoxid und Preußischblau, und organische Farbstoffe, z.B. Alizarin-Farbstoffe, Azofarbstoffe oder Metallphthalocyaninfarbstoffe, und Spurennährstoffe, z.B. Salze von Eisen, Mangan, Bor, Kupfer, Kobalt, Molybdän und Zink, zu verwenden.

Die Präparate enthalten im allgemeinen 0,001 bis 100%, vorzugsweise 0,005 bis 95 Gew.-% der Wirksubstanz.

Die Wirkstoffe können als solche, als ihre Präparate oder als daraus hergestellte Gebrauchsformen, z.B. als gebrauchsfertige Lösungen, Emulsionen, Suspension, Pulver, Pasten und Granulat, verwendet werden. Sie können in üblicher Weise beispielsweise durch Sprühen, Zerstäuben, Stäuben, Streuen und Bewässern angewandt werden.

Es ist auch möglich, die Wirksubstanzen mit Hilfe des ULV-Verfahrens (ultra-low-volume method) anzuwenden, wodurch es möglich ist, die Verbindungen in einer Konzentration bis zu 100% anzuwenden. Die Wirksubstanzen können auch in den Boden eingearbeitet werden.

Im praktischen Gebrauch beträgt der Gehalt an Wirkstoffen in den genannten verschiedenen Formulierungen oder gebrauchsfertigen Präparaten im allgemeinen 0,01 bis 95 Gew.-%, vorzugsweise 0,05 bis 60 Gew.-%.

Die Dosierung oder Aufwandmenge der Wirksubstanz pro Flächeneinheit beträgt im allgemeinen 0,1 bis 10 kg pro ha, vorzugsweise 0,5 bis 3 kg/ha. In besonderen Fällen ist es jedoch möglich oder manchmal sogar notwendig, eine Aufwandmenge außerhalb des vorstehend genannten Bereichs anzuwenden.

Die Erfindung umfaßt ferner ein herbizides Mittel, das als Wirksubstanz eine Verbindung gemäß der Erfindung in Mischung mit einem festen Streckmittel oder Träger oder in Mischung mit einem flüssigen Verdünnungsmittel oder Träger enthält, der ein oberflächenaktives Mittel enthält.

Die Erfindung umfaßt ferner ein Verfahren zur Unkrautbekämpfung, das dadurch gekennzeichnet ist, daß man auf die Unkräuter oder ihren Lebensraum eine Verbindung gemäß der Erfindung

allein oder in Form eines Präparats, das als Wirksubstanz eine Verbindung gemäß der Erfindung in Mischung mit einem Verdünnungsmittel oder Träger enthält, aufbringt.

Die Erfindung ist ferner auf Nutzpflanzen gerichtet, die gegen Schäden durch Unkräuter geschützt sind, indem sie in Bereichen angebaut werden, in denen unmittelbar vor und/oder während der Zeit des Anbaues oder der Entwicklung eine Verbindung gemäß der Erfindung allein oder in Mischung mit einem Streckmittel oder Träger angewandt worden ist.

Es wird ersichtlich sein, daß die üblichen Methoden der Erzeugung von geernteten Nutzpflanzen durch die Erfindung verbessert werden können.

Die Präparate gemäß der Erfindung und die herbizide Wirksamkeit der Verbindungen gemäß der Erfindung werden durch die folgenden Beispiele veranschaulicht. In diesen Beispielen sind die Verbindungen gemäß der Erfindung jeweils durch die (in Klammern genannte) Nummer des entsprechenden Herstellungsbeispiels, das später in dieser Beschreibung folgt, gekennzeichnet.

## Beispiel I

15 Teile der Verbindung (1), 80 Teile eines 1:5-Gemisches von Kieselgurpulver und Tonpulver, 2 Teile Natriumalkylbenzolsulfonat und 3 Teile Natriumalkylnaphthalinsulfonat-Formalin-Kondensat wurden durch Mahlen und Mischen zu einem netzbaren Pulver verarbeitet. Zum Spritzen wurde das Kondensat mit Wasser verdünnt.

## Beispiel II

15 Teile der Verbindung (2), 70 Teile Methyläthylketon, 8 Teile Polyoxyäthylenalkylphenyläther und 7 Teile Calciumalkylbenzolsulfonat wurden durch Mischen und Rühren zu einem emulgierbaren Konzentrat verarbeitet. Zum Spritzen wurde das Konzentrat mit Wasser verdünnt.

## Beispiel III

2 Teile der Verbindung (3) und 98 Teile Tonpulver wurden zur Bildung eines Stäubemittels gemahlen und gemischt.

## Beispiel IV

1,5 Teile der Verbindung (4), 0,5 Teile Isopropylhydrogenphosphit (PAP) und 98 Teile Tonpulver wurden zur Bildung eines Stäubemittels gemahlen und gemischt.

## Beispiel V

10 Teile der Verbindung (5), 30 Teile Bentonit (montmorillonit), 58 Teile Talkum und 2 Teile Ligninsulfonat wurden gemischt. Dem Gemisch wurden 25 Teile Wasser zugesetzt. Das Gemisch wurde innig geknetet und mit einem Extruder-Granulator feinzerteilt, wobei Granulat einer Korngröße von 0,42 bis 2 mm (10-40 mesh), das dann bei 40 bis 50°C getrocknet wurde, erhalten wurde. Das erhaltene Granulat wurde durch Streuen angewandt.

## Beispiel VI

95 Teile Tonteilchen einer Körnung von 0,2 bis 2 mm wurden in einen Drehmischer gegeben. Eine Lösung von 5 Teilen der Verbindung (6) in einem organischen Lösungsmittel wurde während des Drehens über die Teilchen gesprüht, wodurch diese gleichmäßig benetzt wurden. Sie wurden dann bei 40 bis 50°C unter Bildung von Granulat getrocknet. Das Granulat wurde durch Streuen angewandt.

## Beispiel A

Test zur Ermittlung der Wirkung auf Reisfeld-Unkräuter unter wasserüberfluteten Bedingungen (Freilandversuch).

Herstellung der Testverbindung

Unter Verwendung eines Granulators und unter Anwendung eines üblichen Granulierverfahrens wurde das folgende Mittel hergestellt und zu Zylindergranulat von 0,5 mm Durchmesser und 1 bis 3 mm Länge geformt.

Zusammensetzung (insgesamt 100 Teile)

| | |
|---|---|
| Wirkstoff: | 0,8 bis 10 Teile |
| Polyoxyäthylenalkylphenyläther: | 2,5 Teile |
| Bentonit: | 20 Teile |
| Talkum: | 57,5 bis 66,7 Teile |

(Die Talkummenge wurde nach der Menge des Wirkstoffs verändert und die Gesamtmenge hierdurch auf 100 Teile eingestellt.)

Prüfmethoden

1) Chemische Behandlung vor dem Umpflanzen von Reispflanzen

Das Prüffeld wurde unter Verwendung eines Traktors nach üblichen Verfahren des Reisanbaues bis zu einer Tiefe von 20 bis 25 cm gepflügt. Unmittelbar anschließend wurde Wasser von einem Bewässerungskanal in das Feld bis zu einer Höhe von 1 bis 2 cm eingeführt und die erste Bodenbearbeitung (tilling) vorgenommen. Einen oder zwei Tage nach der ersten Bodenbehandlung wurde erneut Wasser bis zu einer Höhe von etwa 5 cm eingeführt und die letzte Bodenbearbeitung durchgeführt. Zwei Tage nach der letzten Bodenbehandlung wurde das Prüffeld in Flächen von je 20 $m^2$ (2 m x 10 m) unter Verwendung starrer Kunststoffplatten unterteilt.

Anschließend wurde das Mittel der vorstehend genannten Zusammensetzung auf das Wasser gegeben. 3 Tage nach der chemischen Behandlung wurden Reissämlinge, die zu diesem Zweck in einer Pflanzschule vorgezogen worden waren (Handelssorte "Kinbae", 15 Tage alt, 2-2,5-Blatt-Phase, Höhe 15 bis 17 cm) unter Verwendung einer motorbetriebenen Reispflanzmaschine umgepflanzt (3 bis 5 Pflanzen pro Wurzel). Vier Wochen nach der chemischen Behandlung wurde die Wirkung der Unkrautbekämpfung nach den nachstehend beschriebenen Merkmalen überprüft.

2) Chemische Behandlung nach dem Umpflanzen der Reispflanzen

Die für die Prüfmethode (1) beschriebenen Maßnahmen bis

zur Stufe der letzten Bodenbehandlung (tilling) wurden befolgt. Zwei Tage nach der letzten Bodenbehandlung wurde die Wassertiefe des Prüffeldes auf 2 bis 3 cm eingestellt, und die Reispflanzen wurden in der gleichen Weise wie bei der Prüfmethode (1) unter Verwendung einer motorbetriebenen Reispflanzmaschine umgepflanzt. Unmittelbar anschliessend wurde das Feld in Flächen von je 20 $m^2$ (2 m x 10 m) unter Verwendung starrer Kunststoffplatten unterteilt, worauf die Wassertiefe des Feldes längere Zeit bei 3 bis 5 cm gehalten wurde. 7 Tage nach dem Umpflanzen der Reispflanzen wurde das Mittel der vorstehend genannten Zusammensetzung auf das Wasser aufgebracht. Vier Wochen nach der chemischen Behandlung wurde die Kontrollwirkung nach dem folgenden Maßstab untersucht:

Die Bewertung der Wirkung erfolgt mit Hilfe der folgenden Skala von 0 bis 5 nach der prozentualen Unkrautvernichtung, bezogen auf die Vernichtung in der unbehandelten Fläche:

5: 95% oder mehr (absolut vollständige Vernichtung)
4: 80% oder mehr, jedoch weniger als 95%
3: 50% oder mehr, jedoch weniger als 80%
2: 30% oder mehr, jedoch weniger als 50%
1: 10% oder mehr, jedoch weniger als 30%
0: weniger als 10% (keine Wirkung)

Die Bewertung "0" für die Phytotoxizität für Reispflanzen bedeutet fehlende Phytotoxizität.

Die Ergebnisse der Versuche sind nachstehend in Tabelle 1 genannt

Tabelle 1

| Verbindung Nr. | Wirkstoffmenge, kg/ha | Behandlung 3 Tage vor Umpflanzung | | | | | | | |
|---|---|---|---|---|---|---|---|---|---|
| | | Bekämpfungswirkung | | | | | | | Phytotoxizität |
| | | Unkraut | | | | | | | Reis |
| | | A | B | C | D | E | F | G | |
| (1) | 1,0 | 5 | 5 | 5 | 5 | 5 | 5 | 5 | 0 |
| (2) | 1,0 | 5 | 5 | 5 | 5 | 5 | 5 | 5 | 0 |
| (3) | 1,0 | 5 | 5 | 5 | 5 | 5 | 5 | 5 | 0 |

Tabelle 1 (Forts.)

| Verbindung Nr. | Wirkstoffmenge, kg/ha | Behandlung 3 Tage vor Umpflanzung | | | | | | | |
|---|---|---|---|---|---|---|---|---|---|
| | | Bekämpfungswirkung | | | | | | | Phytotoxizität |
| | | Unkraut | | | | | | | Reis |
| | | A | B | C | D | E | F | G | |
| (4) | 1,0 | 5 | 5 | 5 | 5 | 5 | 5 | 5 | 0 |
| (5) | 1,0 | 5 | 5 | 5 | 5 | 5 | 5 | 5 | 0 |
| (6) | 1,0 | 5 | 5 | 5 | 5 | 5 | 5 | 5 | 0 |
| (7) | 1,0 | 5 | 5 | 5 | 5 | 5 | 5 | 5 | 0 |
| (8) | 1,0 | 5 | 5 | 5 | 5 | 5 | 5 | 5 | 0 |
| (9) | 1,0 | 5 | 5 | 5 | 5 | 5 | 5 | 5 | 0 |
| (10) | 1,0 | 5 | 5 | 5 | 5 | 5 | 5 | 5 | 0 |
| (11) | 1,0 | 5 | 5 | 5 | 5 | 5 | 5 | 5 | 0 |
| (12) | 1,0 | 5 | 5 | 5 | 5 | 5 | 5 | 5 | 0 |
| (13) | 1,0 | 5 | 5 | 5 | 5 | 5 | 5 | 5 | 0 |
| (14) | 1,0 | 5 | 5 | 5 | 5 | 5 | 5 | 5 | 0 |
| (15) | 1,0 | 5 | 5 | 5 | 5 | 5 | 5 | 5 | 0 |
| (16) | 1,0 | 5 | 5 | 5 | 5 | 5 | 5 | 5 | 0 |
| (17) | 1,0 | 5 | 5 | 5 | 5 | 5 | 5 | 5 | 0 |
| (18) | 1,0 | 5 | 5 | 5 | 5 | 5 | 5 | 5 | 0 |
| (19) | 1,0 | 5 | 5 | 5 | 5 | 5 | 5 | 5 | 0 |
| (20) | 1,0 | 5 | 5 | 5 | 5 | 5 | 5 | 5 | 0 |
| IV-1 | 1,0 | 3 | 4 | 3 | 2 | 0 | 4 | 2 | 0 |
| | | Behandlung 7 Tage nach Umpflanzung | | | | | | | |
| (1) | 1,0 | 5 | 5 | 5 | 5 | 5 | 5 | 5 | 0 |
| (2) | 1,0 | 5 | 5 | 5 | 5 | 5 | 5 | 5 | 0 |
| (3) | 1,0 | 5 | 5 | 5 | 5 | 5 | 5 | 5 | 0 |
| (4) | 1,0 | 5 | 5 | 5 | 5 | 5 | 5 | 5 | 0 |
| (5) | 1,0 | 5 | 5 | 5 | 5 | 5 | 5 | 5 | 0 |
| (6) | 1,0 | 5 | 5 | 5 | 5 | 5 | 5 | 5 | 0 |
| (7) | 1,0 | 5 | 5 | 5 | 5 | 5 | 5 | 5 | 0 |
| (8) | 1,0 | 5 | 5 | 5 | 5 | 5 | 5 | 5 | 0 |
| (9) | 1,0 | 5 | 5 | 5 | 5 | 5 | 5 | 5 | 0 |
| (10) | 1,0 | 5 | 5 | 5 | 5 | 5 | 5 | 5 | 0 |
| (11) | 1,0 | 5 | 5 | 5 | 5 | 5 | 5 | 5 | 0 |
| (12) | 1,0 | 5 | 5 | 5 | 5 | 5 | 5 | 5 | 0 |
| (13) | 1,0 | 5 | 5 | 5 | 5 | 5 | 5 | 5 | 0 |

Tabelle 1 (Forts.)

| Verbindung Nr. | Wirkstoffmenge kg/ha | Behandlung 7 Tage nach Umpflanzung | | | | | | | |
|---|---|---|---|---|---|---|---|---|---|
| | | Bekämpfungswirkung | | | | | | | Phytotoxizität |
| | | Unkraut | | | | | | | Reis |
| | | A | B | C | D | E | F | G | |
| (14) | 1,0 | 5 | 5 | 5 | 5 | 5 | 5 | 5 | 0 |
| (15) | 1,0 | 5 | 5 | 5 | 5 | 5 | 5 | 5 | 0 |
| (16) | 1,0 | 5 | 5 | 5 | 5 | 5 | 5 | 5 | 0 |
| (17) | 1,0 | 5 | 5 | 5 | 5 | 5 | 5 | 5 | 0 |
| (18) | 1,0 | 5 | 5 | 5 | 5 | 5 | 5 | 5 | 0 |
| (19) | 1,0 | 5 | 5 | 5 | 5 | 5 | 5 | 5 | 0 |
| (20) | 1,0 | 5 | 5 | 5 | 5 | 5 | 5 | 5 | 0 |
| IV-1 | 1,0 | 3 | 3 | 3 | 2 | 0 | 4 | 1 | 0 |

Bemerkungen

1) Die Buchstaben A, B, C, D, E, F und G in der Spalte mit der Überschrift "Unkraut" stellen die folgenden Unkräuter dar:

A: Panicum crus-galli

B: Scirpus juncoides Roxburgh var.

C: Monochoria vaginalis Presl

D: Breitblättrige Unkräuter (Lindernia Procumbens Philcox, Rotala indica Koehne, Elatine triandra Schk usw.)

E: Sagittaria pygmaea Miq.

F: Eleocharis acicularis L.

G: Cyperus serotinus Rottboell

2) Die Vergleichsverbindung IV-1 hatte die Formel

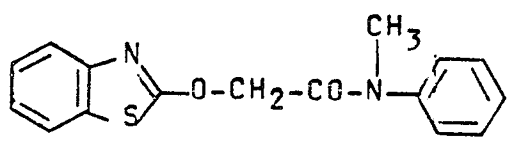

(siehe japanische Auslegeschrift 154762/1979).

Das Verfahren zur Herstellung der Verbindungen gemäß der Erfindung wird durch die folgenden Herstellungsbeispiele veranschaulicht.

## Beispiel 1

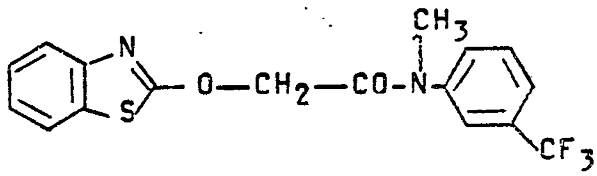

(1)

1,7 g 2-Chlorbenzothiazol wurden tropfenweise zu einer wäßrigen Lösung von 0,75 g Kaliumhydroxidpulver und 2,6 g 3'-Trifluormethyl-N-methylhydroxyacetanilid gegeben, während die Temperatur bei 20°C gehalten wurde. Das Reaktionsgemisch wurde auf 40°C erhitzt und 2 Stunden bei dieser Temperatur gerührt. Das Reaktionsgemisch wurde der Abkühlung überlassen und in Wasser gegossen. Die ausgefällten Kristalle wurden abfiltriert und aus Äthanol/Wasser umkristallisiert, wobei 3,0 g des gewünschten Produkts, Benzothiazol-2-yloxyessigsäure-N-methyl-3-trifluormethylanilid, in Form von weißen Kristallen vom Schmelzpunkt 128 - 129°C erhalten wurden.

In analoger Weise wurden die in Tabelle 2 genannten Verbindungen gemäß der Erfindung hergestellt.

Tabelle 2

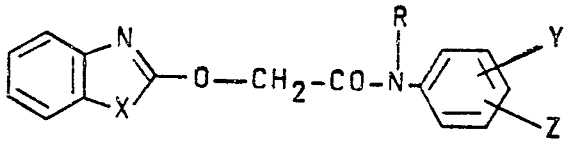

(I)

| Beispiel Nr. | X | R | Y | Z | Physikalische Konstante, Schmelzpunkt (°C) |
|---|---|---|---|---|---|
| 2 | O | $CH_3$ | $2-CF_3$ | H | 130 - 132 |
| 3 | S | $CH_3$ | $2-CF_3$ | H | 135 - 137,5 |
| 4 | O | $CH_3$ | $3-CF_3$ | H | 125 - 130 |
| 5 | O | $CH_3$ | $4-CF_3$ | H | 103 - 107 |
| 6 | S | $CH_3$ | $4-CF_3$ | H | 112 - 117 |
| 7 | O | $CH_3$ | $3-CF_3$ | $5-CF_3$ | 138 - 139 |
| 8 | S | $CH_3$ | $3-CF_3$ | $5-CF_3$ | 119 - 121 |
| 9 | O | $CH_3$ | $2-O-CH_3$ | H | 164 - 166 |
| 10 | S | $CH_3$ | $2-O-CH_3$ | H | 125 - 130 |
| 11 | O | $CH_3$ | $3-O-CH_3$ | H | 123,5 - 125 |
| 12 | S | $CH_3$ | $3-O-CH_3$ | H | 104 - 105 |
| 13 | O | $CH_3$ | $4-O-CH_3$ | H | 137 - 142 |
| 14 | S | $CH_3$ | $4-O-CH_3$ | H | 113 - 114 |
| 15 | O | $CH_3$ | $3-O-C_3H_7$-iso | H | 119 - 120 |
| 16 | S | $CH_3$ | $3-O-C_3H_7$-iso | H | 84 - 85 |
| 17 | O | $CH_3$ | 3-O- 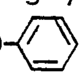 | H | 105 - 106 |
| 18 | S | $CH_3$ | 3-O- 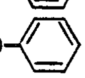 | H | 122 - 124 |
| 19 | 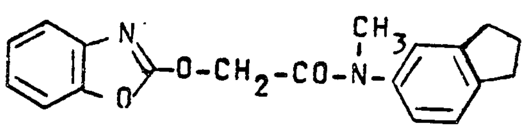 | | | | 112,5 - 114 |
| 20 | 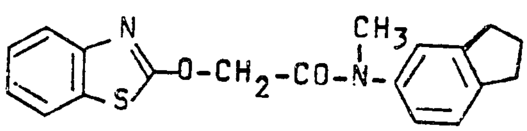 | | | | 141 - 142 |

Patentansprüche:

1. Substituierte Acetanilide der allgemeinen Formel

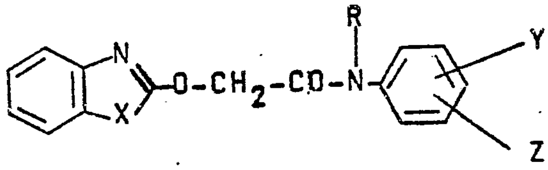

(I)

in der X ein Sauerstoffatom oder ein Schwefelatom ist, R ein Alkylrest mit 1 bis 4 C-Atomen ist und Y und Z, die gleich oder verschieden sein können, jeweils für ein Wasserstoffatom, einen Alkoxyrest mit 1 bis 4 C-Atomen, einen Halogenalkylrest oder einen Phenoxyrest stehen oder gemeinsam eine Trimethylengruppe bilden mit der Maßgabe, daß Y und Z nicht beide Wasserstoffatome sind.

2. Verbindungen nach Anspruch 1, dadurch gekennzeichnet, daß Y und Z jeweils für ein Wasserstoffatom, einen Methoxyrest, Äthoxyrest, einen n-Propoxy- oder Isopropoxyrest oder einen n-Butoxy-, Isobutoxy-, sek.-Butoxy- oder t-Butoxyrest, einen Phenoxyrest oder einen Monohalogenmethyl-, Monohalogenäthyl-, Dihalogenmethyl-, Dihalogenäthyl-, Trihalogenmethyl- oder Trihalogenäthylrest stehen, wobei der Halogensubstituent bzw. die Halogensubstituenten Fluor, Chlor, Brom oder Jod sind, oder Y und Z gemeinsam eine Trimethylengruppe bilden.

3. Verfahren zur Herstellung von substituierten Acetaniliden nach Anspruch 1 oder 2, dadurch gekennzeichnet, daß man ein 2-Halogenbenzazol der allgemeinen Formel

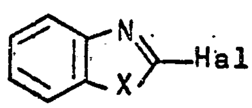

(II),

in der X die in Anspruch 1 genannte Bedeutung hat und Hal ein Halogenatom ist, mit einer Verbindung der allgemeinen Formel

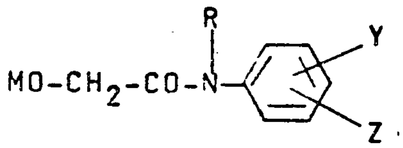 (III),

in der R, Y und Z die in Anspruch 1 genannten Bedeutungen haben und M ein Wasserstoffatom oder ein Alkalimetallatom ist, gegebenenfalls in Gegenwart eines Verdünnungsmittels und gegebenenfalls in Gegenwart eines Säureakzeptors.

4. Herbizide Mittel, gekennzeichnet durch einen Gehalt an einem substituierten Acetanilid der Formel (I) gemäß Anspruch 1.

5. Verfahren zur Bekämpfung von unerwünschtem Pflanzenwachstum, dadurch gekennzeichnet, daß man ein substituiertes Acetanilid der Formel (I) gemäß Anspruch 1 auf die unerwünschten Pflanzen oder ihren Lebensraum einwirken läßt.

6. Verwendung von substituierten Acetaniliden der Formel (I) gemäß Anspruch 1 zur Bekämpfung von Unkraut.

7. Verfahren zur Herstellung von herbiziden Mitteln, dadurch gekennzeichnet, daß man substituierte Acetanilide der Formel (I) gemäß Anspruch 1 mit Streckmitteln und/oder oberflächenaktiven Mitteln vermischt.

Europäisches Patentamt

# EUROPÄISCHER RECHERCHENBERICHT

0037938

Nummer der Anmeldung

EP 81 10 2277

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | EP - A - 0 005 501 (BAYER A.G.)<br>* Ansprüche; Seiten 22-37 *<br>-- | 1-7 |
| P | EP - A - 0 018 497 (BAYER A.G.)<br>* Ansprüche 1-6 *<br>---- | 1,3-7 |

KLASSIFIKATION DER ANMELDUNG (Int. Cl.)

C 07 D 263/58
277/68
A 01 N 43/76
43/78

RECHERCHIERTE SACHGEBIETE (Int. Cl.)

C 07 D 263/58
277/68
A 01 N 43/74
43/76
43/78

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsatze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 24-06-1981 | HENRY |

EPA form 1503.1 06.78